(19) Europäisches Patentamt
European Patent Office
Office européen des brevets

(11) Publication number: **0 415 567 A2**

# EUROPEAN PATENT APPLICATION

(21) Application number: 90308442.4

(51) Int. Cl.5: **A61K 47/02**

(22) Date of filing: **31.07.90**

| | |
|---|---|
| The title of the invention has been amended (Guidelines for Examination in the EPO, A-III, 7.3). | (71) Applicant: **QUADRANT BIORESOURCES LIMITED**<br>**Cambridge Research Laboratories, 181A Huntingdon Road**<br>**Cambridge, CB3 ODJ(GB)** |
| (30) Priority: **31.07.89 GB 8917470** | |
| (43) Date of publication of application:<br>**06.03.91 Bulletin 91/10** | (72) Inventor: **Roser, Bruce Joseph**<br>**The Old Vicerage Church Lane**<br>**Balsham, Cambridgeshire(GB)** |
| (84) Designated Contracting States:<br>**AT BE CH DE DK ES FR GB GR IT LI LU NL SE** | (74) Representative: **Ablewhite, Alan James et al**<br>**MARKS & CLERK 57/60 Lincoln's Inn Fields**<br>**London WC2A 3LS(GB)** |

(54) Composition and method for stabilising organic compounds.

(57) Preservation of water-sensitive organic compounds comprises intimately mixing the compound with an efflorescent salt. A stabilised composition is obtained comprising a stabilised composition comprising an intimate mixture of a hygroscopic or moisture sensitive low-molecular weight organic compound and an efflorescent salt.

EP 0 415 567 A2

## COMPOSITION AND METHOD

This invention relates to a method for preventing degradation of moisture-sensitive low-molecular weight compounds, for example antibiotics; and to stabilised compositions.

Very many organic compounds are moisture-sensitive and must be stored under dry conditions if their intrinsic activity or utility is to be maintained. This means that the compounds are unstable in aqueous systems and must be stored as dry powders. However. ensuring a constant low moisture content in dry formulations can be a problem if the compound itself is hygroscopic. In such cases it is necessary for the dry material to be sealed into water-impervious foil and plastic sachets, which must remain sealed until the material is about to be used. An additional problem is that many such substances are freeze- dried (lyophilised) in order to improve eventual water solubility and the finely divided product so obtained is particularly hygroscopic. Spray drying is also a commonly used process and also leads to a finely divided product.

For example, Ampicillin sodium is defined in Martindale The Extra Pharmacopoeia as being hygroscopic and absorbing substantial amounts of moisture even at low relative humidities. The stability of solutions of Ampicillin sodium in water is dependant on the concentration, the nature of the vehicle and the temperature. The stability decreases as the concentration increases. Thus. a hygroscopic powder that absorbs moisture will produce a very highly concentrated viscous paste which is particularly unstable since solutions of 25% wv lose 20% of their potency in six hours at 5°C (Martindale, ibid). The problem is made worse if the dried substance contains buffer materials commonly used in injection preparations, such phosphate buffered saline (PBS). Another example involves intercollating and labelling dyestuffs and indicators, such as Acridine Orange, while a third example is the complexing agent EDTA.

There is thus a need for a way of producing dry material which is not hygroscopic and is substantially more stable to ambient atmospheric conditions, but which nevertheless retains its solubility.

We have now found that low-molecular weight organic compounds such as antibiotics and other antimicrobials complexing agents, dyestuffs etc, and dried preparations thereof (e.g. bulk powders. and formulated powders for eventual redissolving for administration) can be stabilised against moisture-induced degradation by being intimately mixed with an efflorescent salt, in particular a salt which has a vapour pressure of crystallisation of at least 15mm Hg (2000 Pa). Efflorescent is defined in Dorland's Illustrated Medical Dictionary as: becoming powdery in consequence of losing water of crystallisation. The intimate mixing is conveniently achieved by co-drying a solution or suspension of the organic compound which also contains the efflorescent salt. The efflorescent salt may comprise a buffer system, or can effectively replace a substantial amount of a non-efflorescent buffer in a conventional formulation.

The drying of the system can conveniently be ach#eved by freeze-drying, but ambient or elevated temperature drying may also be possible (including spray drying). depending on the nature of the organic compound being stabilised. The organic compound is referred to herein as a low molecular weight compound in order to indicate that it is not a macromolecular biological substance such as an enzyme, virus, antibody etcetera. Typically, molecular weights of less than 1500, e.g. between 100 and 750, are envisaged.

Depending on the field of use of the organic compound being stabilised, the choice of efflorescent salt can be made among any of those which are suitable for pharmaceutical or food use or which will otherwise be compatible with the end use of the compound. Typical sodium salts include the acetate trihydrate, tetraborate decahydrate (Borax), bromoiridite dodecahydrate. carbonate heptahydrate metaperiodate trihydrate, metaphosphate hexahydrate hydrogen orthophosphate dodecahydrate, sulphite heptahydrate. thiosulphate pentahydrate (hypo). Possible non-sodium salts include calcium lactate hydrate, magnesium salicylate tetrahydrate, magnesium sulphate heptahydrate (Epsom salts) and ammonium sulphate. The amount of salt present in the dried composition can vary over a wide range. Generally speaking, any amount of efflorescent salt above zero will have some effect, while high levels of salt may be undesirable in the rehydrated formulation. Typically a ratio of salt to organic compound of from 1:5 to 1:75, typically 1: 20 to 1:50, e.g. 1:25 to 1:40, is applicable.

We have also found it desirable to incorporate in the co-dried material a sugar, particularly trehalose. Certain sugars, particularly trehalose, are known as having a stabilising effect on biological macromolecules undergoing drying. For example PCT/GB86/00396 describes the use of trehalose to stabilise biological materials against drying at ambient or even elevated temperatures. However. it would not be expected that low molecular weight organic compounds such as antibiotics would need the kind of stabilisation required for delicate proteins. Nevertheless. we have found that inclusion of the sugar in the composition and method of this invention has the surprisingly beneficial effect of greatly increasing the speed and ease of the dissolution of the product. Thus. in a preferred embodiment of the invention, the organic compound is stabilised in the dry state by being intimately admixed with both an efflorescent salt and a sugar, particularly trehalose.

The following examples illustrate the invention.

Example 1

Solutions of Ampicillin sodium containing 0.5g antibiotic per ml of buffer solution were freeze-dried and the dried product was stored for one week at room temperature at 50% relative humidity in an open container. The dried material was then re-dissolved and tested for activity against Ampicillin-sensitive microorganisms on sensitivity plates.

Solution 1 - phosphate-buffered saline pH 7.4 + 0.3M trehalose

The dried material after storage was caked and showed negligible activity.

Solution 2 - 100 mM sodium sulphate + 10 mM Tris HCl buffer, pH 7.4 + 0.3 M trehalose

The dried material remained a free flowing dry powder after storage with the same degree of activity as a fresh sample or a sample stored under chilled dry conditions. The material rapidly imbibed added water to form a clear solution. An equivalent material containing sodium sulphate but no trehalose also remained dry and active, but formed a water-repellent powder which took over ten minutes to form a clear solution.

Example 2

An antiglycophorin antibody (Ab) was dried in the presence of trehalose (10% w/v), and in some cases with Acridine Orange (AO 6mM final) in addition to sodium sulphate ($Na_2SO_4$ 200mM final), stored at room temperature, and assayed for its ability to regulate red blood cells.

| Results | Functional test carried out after 39 days storage |
|---|---|
| | % of control activity |
| Ab T AO | 85% |
| Ab T AO $Na_2SO_4$ | 100% |
| AB T | 100% |

The results show that
1. In the presence of trehalose alone, the antibody activity is maintained.
2. On the addition of Acridine Orange to this mixture, there is a loss of activity.
3. This loss of activity is due to the hygroscopic nature of Acridine Orange which can be alleviated by the addition of an efflorescent salt (in this case, N sodium sulphate).

Example 3

Samples containing 280 mm ethylene diamine tetra-acetic acid (EDTA) 0.07% (v/v), polyoxyethylene sorbitan monolaurate (Tween 80) 10% w/v trehalose and in some cases, where indicated, 500 mM sodium sulphate, were dried and then stored at a relative humidity of 90% for 6 days at ambient temperature for gravimetric analysis.

3

| Results | % weight change after 6 days storage at 90% R.M. |
|---|---|
| EDTA Tween 80 Trehalose | +22% |
| EDTA Tween 80 Trehalose $Na_2SO_4$ | +17.7% |

The presence of an efflorescent salt (sodium sulphate) had reduced the hygroscopic nature of the compounds.

Example 4

Storage of ATP in trehalose:-

Using an assay for DNA ligase whioh joins DNA fragments using ATP as an energy source. ATP at 0.5 mM dried with ligase (in the buffer in the high temperature storage patent i.e. no acetate as an efflorescent salt) is completely stable for 2 weeks at 37°C in the dry state.

**Claims**

1. A method of obtaining a dry, non hygroscopic preparation of a hygroscopic or moisture-sensitive low-molecular weight organic compound comprising intimately mixing the compound with an efflorescent salt.

2. A method according to Claim 1, in which the intimate mixing is effected by drying an aqueous co-solution or suspension of the compound and the efflorescent salt.

3. A method according to Claim 1 or Claim 2, in which the efflorescent salt comprises all or a substantial part of a buffer system.

4. A method according to any of Claims 1 to 3 in which the compound is selected from an antibiotic, a dyestuff and a complexing agent.

5. A method according to any of CLaims 1 to 4 in which the efflorescent salt is selected from sodium acetate trihydrate, tetraborate, decahydrate (Borax), bromoiridite dodecahydrate, carbonate heptahydrate, metaperiodate trihydrate, metaphosphate hexahydrate, hydrogen orthophosphate dodecahydrate, sulphite heptahydrate, thiosulphate pentahydrate, calcium lactate hydrate, magnesium salicylate tetrahydrate, magnesium sulphate heptahydrate (epsom salts) and ammonium sulphate.

6. A method according to any of Claims 1 to 5, in which the ratio of salt to organic compound is from 1:5 to 1:75 by weight.

7. A method according to any of Claims 1 to 6, in which a sugar is also incorporated in the mixture.

8. A method according to Claim 7, in which the sugar is trehalose.

9. A stabilised composition comprising an intimate mixture of a hygroscopic or moisture sensitive low-molecular weight organic compound and an efflorescent salt.

10. A composition according to Claim 9 in which the organic compound is selected from an antibiotic, a dyestuff and a complexing agent.

11. A composition according to Claim 9 or Claim 10 in which the ratio of salt to organic compound is from 1:5 to 1:75 by weight.

12. A composition according to any of Claims 9 to 11 which the salt is selected from the group consisting of sodium acetate trihydrate, tetraborate, decahydrate (Borax), bromoiridite dodecahydrate, carbonate heptahydrate, metaperiodate trihydrate, metaphosphate hexahydrate, hydrogen orthophosphate dodecahydrate, sulphite heptahydrate, thiosulphate pentahydrate, calcium lactate hydrate, magnesium salicylate tetrahydrate, magnesium sulphate heptahydrate (epsom salts) and ammonium sulphate.

13. A composition according to any of Claims 9 to 12 further containing a sugar.

14. A composition according to Claim 13, in which the sugar is trehalose.